(19) Europäisches Patentamt
European Patent Office
Office européen des brevets

(11) Numéro de publication: **0 153 223**
**B1**

(12) # FASCICULE DE BREVET EUROPÉEN

(45) Date de publication du fascicule du brevet: **17.12.86**

(51) Int. Cl.⁴: **C 07 C 143/46**

(21) Numéro de dépôt: **85400209.4**

(22) Date de dépôt: **08.02.85**

(54) **Procédé de préparation de para acyloxybenzènesulfonates par catalyse basique.**

(30) Priorité: **17.02.84 FR 8402399**

(43) Date de publication de la demande: **28.08.85 Bulletin 85/35**

(45) Mention de la délivrance du brevet: **17.12.86 Bulletin 86/51**

(84) Etats contractants désignés: **AT BE CH DE FR GB IT LI LU NL SE**

(56) Documents cité:
**EP-A-0 105 673**
**FR-A-2 299 321**

(73) Titulaire: **RHONE- POULENC CHIMIE DE BASE, 25, quai Paul Doumer, F-92408 Courbevoie (FR)**

(72) Inventeur: **Moyne, José, 4, Chemin de la Vire, F-69300 - Caluire (FR)**
Inventeur: **Disdier, Camille, 12, rue Barodet, F-69004 - Lyon (FR)**

(74) Mandataire: **Dubruc, Philippe, RHONE- POULENC INTERSERVICES Service Brevets Chimie 25, quai Paul- Doumer, F-92408 Courbevoie Cédex (FR)**

**EP 0 153 223 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1986

**Description**

La présente invention concerne un procédé de préparation de para aciloxybenzènesulfonates. Elle concerne plus particulièrement la préparation par catalyse basique de para acyloxybenzènesulfonates dans lesquels le groupe acyloxy contient entre 7 et 12 atomes de carbone.

Il est connu d'après le brevet français N° 2 164 619 exemple 1 de fabriquer ces composés à partir d'un chlorure d'acide aliphatique et du phénolsulfonate de potassium par condensation directe en milieu anhydre. La vitesse de condensation entre le chlorure d'acide et le phénolsulfonate est extrêmement lente (20 heures à 150° C) et le produit formé est très difficile à isoler. Il y a aussi dans ce procédé une formation importante d'HCl dont l'élimination n'est pas toujours aisée.

Il est aussi connu -PUESCHEL, Tenside 7 (5) p. 249-54 (1970)-de fabriquer ces composés selon un procédé peu différent du brevet français N° 2 164 619 mais en présence d'un accepteur d'acide pour éviter l'élimination d'acide chlorhydrique gazeux. Le produit formé est neutralisé par un carbonate, de sodium, mais il est alors très difficile de séparer le produit obtenu du chlorure de sodium formé lors de la neutralisation.

La lenteur de la réaction de condensation du chlorure d'acide sur le phénolsulfonate a amené l'homme de l'art à augmenter considérablement la température mais il y a alors formation de produits fortement colorés. Ces produits étant en effet utilisés pour la plupart en détergence, il est nécessaire de disposer de produits parfaitement blancs pour répondre aux exigences commerciales.

Il est également connu d'après le brevet français N° 2 299 321 de fabriquer des para acyloxybenzènesulfonates par condensation d'une poudre de phénolsulfonate avec de l'anhydride acétique sous forme de vapeur; cette réaction est réalisable à sec car l'anhydride acétique a un point d'ébullition de 140° C mais il est inenvisageable de procéder de cette manière pour condenser par exemple l'anhydride nonanoïque sur le phénolsulfonate car l'anhydride nonanoïque a un point d'ébullition de 260° C.

Aucune publication décrivant la condensation d'un anhydride d'acide et d'un phénolsulfonate en milieu liquide n'est connue.

Il a maintenant été découvert un procédé permettant de vaincre tous ces inconvénients, c'est-à-dire permettant d'obtenir une réaction rapide en milieu liquide, une séparation facile du produit de condensation et l'obtention de produits ton colorés.

Ce procédé de préparation de para acyloxybenzènesulfonates de formule générale suivante:

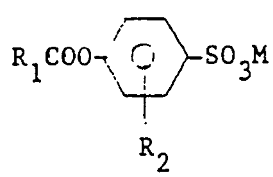 (I)

dans laquelle:

$R_1$ est un radical aliphatique, contenant de 6 à 11 atomes de carbone, linéaire ou ramifié;

$R_2$ est un radical choisi parmi le groupe comprenant l'hydrogène, les halogènes, les radicaux alkyles ayant de 1 à 4 atomes de carbone, le radical $SO_3M$.

M est un métal alcalin, alcalino-terreux ou un groupe ammonium est caractérisé en ce qu'on acyle un phénolsulfonate alcalin, alcalino-terreux ou d'ammonium par l'anhydride d'un acide contenant 7 à 12 atomes de carbone, linéaire ou ramifié, dans un solvant aprotique polaire en présence d'une quantité catalytique d'un sel alcalin ou alcalinoterreux d'un acide aliphatique linéaire ou ramifié ayant de 7 à 12 atomes de carbone.

On peut citer parmi les anhydrides aliphatiques pouvant être mis en oeuvre, les anhydrides heptanoïque, octanoïque, caprylique, nonanoïque, pelargonique, decanoïque, caprique, dodecanoïque, laurique. La présente invention est particulièrement bien adaptée aux anhydrides d'acides contenant neuf atomes de carbone. Parmi ces anhydrides sont particulièrement concernés les anhydrides pelargonique et 3,5,5 triméthylhexanoïque car ils sont disponibles industriellement.

Les anhydrides d'acide peuvent être préparés de manière connue par plusieurs procédés. Selon une première forme de mise en oeuvre décrite dans "Organic synthèse collective" John Wiley, 1955 volume 3 p. 28, on peut mettre en contact le chlorure d'acide, l'acide et une base tertiaire qui va neutraliser l'acide formé. On obtient ainsi l'anhydride recherché et un chlorhydrate de base tertiaire. Selon une deuxième forme de mise en oeuvre décrite dans "Journal of Chemical Society" 1964 p. 755, on peut mettre en contact le chlorure d'acide et le sel de sodium de l'acide dans l'eau. On obtient ainsi l'anhydride recherché et du chlorure de sodium. Cette réaction ayant lieu dans l'eau, l'anhydride formé ne doit pas être facilement hydrolysable.

Selon une troisième forme de mise en oeuvre, on fait réagir l'anhydride acétique avec l'acide selon le mécanisme suivant

$$2RCOOH + (CH_3\,CO)_2O \xrightarrow[CH_3\,COONa]{} (RCO)_2\,O + 2CH_3COOH$$

On préfère opérer en présence d'un excès d'anhydride acétique qui est distillé en fin de réaction.

Selon le procédé de l'invention, on préfère utiliser un anhydride d'acide obtenu selon cette troisième forme de mise en oeuvre.

Parmi les différents phénolsulfonates on préfère utiliser les phénolsulfonates dans lesquels $R_2$ est l'hydrogène et tout particulièrement le phénol sulfonate de sodium et de potassium car ils sont ceux qui industriellement sont le plus facilement disponibles.

Parmi les solvants aprotiques polaires on peut citer par exemple:
- le diméthylformamide
- la N méthylpyrrolidone
- le diméthylacétamide
- le diméthylsulfoxide
- le sulfolane

Le solvant doit néanmoins être non odorant car il est impossible d'un point de vue commercial d'incorporer dans un mélange lessiviel un produit malodorant. Le solvant doit avoir un point d'ébullition pas trop élevé et il doit être d'un prix de revient suffisamment bas pour ne pas grever inutilement le prix du produit à obtenir. Le diméthylformamide est parmi tous ces solvants celui qui répond le mieux à ces conditions.

Le sel alcalin ou alcalinoterreux d'un acide ayant 7 à 12 atomes de carbone utilisé comme catalyseur de la réaction de condensation répond à la formule générale suivante:

$$R_3COO\ M \qquad (II)$$

dans laquelle: M est un métal alcalin ou alcalinoterreux $R_3$ est un radical alkyle linéaire ou ramifié contenant 6 à 11 atomes de carbone. On utilise de préférence le sel de sodium du même acide que celui qui sert à former l'anhydride ce qui permet d'éviter l'incorporation d'éléments chimiques étrangers à ceux de la réaction.

Pour obtenir une vitesse de réaction correcte, il est préférable de mettre un excès molaire d'anhydride par rapport au phénolsulfonate. Pour un rendement économique correct, il est encore plus préférable d'en ajouter un excès d'au moins 0,2 mole et de préférence compris entre 0,2 et 0,3 mole par rapport à la stoechiométrie de la réaction.

Le rapport molaire du solvant au phénolsulfonate est de préférence compris entre 5 et 50. Une quantité supérieure n'est pas exclue du domaine de l'invention, mais il faudra adapter les quantités à l'économie du procédé; plus préférentiellement, ce rapport molaire est compris entre 5 et 10 et encore plus préférentiellement ce rapport est compris entre 7 et 10.

Le rapport molaire du composé (II) au phénol sulfonate est de préférence supérieur à environ 0,005 et préférentiellement compris entre 0,01 et 0,02.

La température de réaction influe sur la vitesse de réaction ainsi une température supérieure à 80°C est avantageuse. Mais une température supérieure n'est pas nuisible au procédé de l'invention. Il faudra seulement adapter la température à l'économie du procédé. La température préférentielle de réaction se situe donc entre 90 et 100°C.

La réaction est généralement conduite à pression atmosphérique bien qu'une pression supérieure ne soit pas nuisible au procédé de l'invention.

Les produits selon l'invention peuvent être extraits du milieu réactionnel par relargage à l'acétone, à une température égale ou supérieure à 90°C et de préférence comprise entre 90 et 100°C en ajoutant environ le même poids d'acétone que celui du solvant introduit.

Les para acyloxybenzènesulfonates sont utilisés en détergence comme substances tensioactives. On peut citer comme exemples de composés de formule (I): le triméthyl-3,5,5 hexanoyloxy-benzènesulfonate de sodium, l'octanoylbenzènesulfonate de sodium, le dodecanoyloxybenzènesulfonate de sodium.

L'invention va être plus complètement décrite à l'aide des exemples qui vont suivre.

**Exemple 1**

Triméthyl-3,5,5 hexanoyloxy-benzènesulfonate de sodium

1 - Anhydride triméthyl-3,5,5, hexanoïque (anhydride T.M.H.)

Dans un réacteur de 1.000 litres, surmonté d'une colonne à distiller, on charge 583 kg d'acide triméthylhexanoïque (3,69 Kmoles), 282 kg d'anhydride acétique (2,76 Kmoles) et 0,1 kg d'acétate de sodium. Ce catalyseur a été choisi compte tenu de la faible coloration qu'il donne à l'anhydride T.M.H. Le milieu réactionnel est porté à 90°C sous un vide de 12 000 Pa pour permettre la distillation de l'acide acétique formé; le vide est ensuite ajusté à mesure que la température du bouilleur monte.

Après 3 heures, la réaction est terminée: T°: 110°C p = 6 660 Pa

Le milieu réactionnel est porté à 160°C (sous 1 300 Pa) pour éliminer l'excès d'anhydride acétique.

L'anhydride triméthyl-3,5,5 hexanoïque très faiblement coloré n'est pas distillé mais utilisé tel quel: p = 550 kg R = 100 %

2 - Condensation de l'anhydride T.M.H. sur le p.phénolsulfonate de sodium

Dans un réacteur de 3 $m^3$ surmonté d'une petite colonne, on introduit 794 kg (10,9 kmoles) de diméthylformamide, 301 kg (1,53 kmole) de p-phénolsulfonate de sodium séché à 160°C sous 2 600 Pa ($H_2O$ < 0,5 %) et 3 kg (0,016 kmole) d'isononanoate de sodium.

Le milieu réactionnel est porté à 90°C et 550 kg (1,84 kmole) d'anhydride T.M.H (20 % excès) sont introduits en une demi-heure à trois quart d'heure.

La température est maintenue 3 heures.

On introduit à une température de 90 à 100°C, 794 kg d'acétone pour relarguer l'ester qui est en solution dans le DMF.

On refroidit jusqu'à température ambiante.

L'ester est filtré sur un filtre de 6 $m^2$ de surface travaillant sous pression.

Le produit filtré est lavé avec de l'acétone et séché à 150°C sous 2 600 Pa.

p = 500 kg R = 96 %

Les solutions sont distillées et recyclées.

**Exemple 2**

Ethyl-2 hexyloxy-benzènesulfonate de sodium

1 - Anhydride Ethyl-2 hexanoïque

L'anhydride éthyl-2 hexanoïque est réalisé dans les mêmes conditions que dans l'exemple précédent.

2 - Condensation de l'anhydride éthyl-2 hexanoïque sur le p.phénolsulfonate de sodium

Dans un réacteur de 250 $cm^3$ sont introduits 66 g (0,9 mole) de DMF, 25 g (0,127 mole) de para phénolsulfonate de sodium déhydraté et 0,35 g (0,002 mole) d'éthyl-2 hexanoate de sodium.

Le milieu réactionnel est porté à 100°C, on ajoute en 45 minutes 45 g (0,160 mole) soit 25 % d'excès d'anhydride éthyl-2 hexanoïque;

La réaction est maintenue 2 h 30 à cette température.

On ajoute ensuite 80 g d'acétone à une température de 100°C.

On refroidit jusqu'à température ambiante, puis on filtre le produit.

L'ester est lavé à l'acétone et séché à 150°C sous 2600 Pa.

On obtient:

p = 39 g d'éthyl)2 hexyloxy-benzènesulfonate de sodium R = 95 %

**Revendications**

1. Procédé de préparation de para acyloxybenzènesulfonates de formule générale suivante:

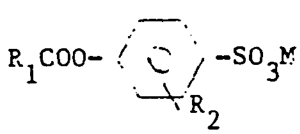 dans laquelle :

- $R_1$ est un radical aliphatique contenant 6 à 11 atomes de carbone linéaire ou ramifié, - $R_2$ est un radical choisi parmi le groupe comprenant l'hydrogène, les halogènes, les radicaux alkyles ayant de 1 à 4 atomes de carbone, le radical $SO_3M$, - M est un métal alcalin, alcalino-terreux, un groupe ammonium caractérisé en ce qu'on acyle un phénol sulfonate alcalin, alcalinoterreux ou d'ammonium par l'anhydride d'un acide contenant 7 à 12 atomes de carbone, linéaire ou ramifié dans un solvant aprotique polaire en présence d'une quantité catalytique d'un sel alcalin ou alcalinoterreux d'un acide aliphatique linéaire ou ramifié contenant 7 à 12 atomes de carbone.

2. Procédé selon la revendication 1 caractérisé en ce que le phénol sulfonate est le phénol sulfonate de sodium ou de potassium.

3. Procédé selon la revendication 1 caractérisé en ce que l'anhydride d'acide est l'anhydride d'un acide contenant 9 atomes de carbone.

4. Procédé selon la revendication 1 caractérisé en ce que le solvant aprotique polaire est choisi parmi le diméthylformamide, le diméthylacétamide, la N-méthyl pyrrolidone, le diméthylsulfoxide et le sulfolane.

5. Procédé selon la revendication 5 caractérisé en ce que le solvant aprotique polaire est le diméthylformamide.

6. Procédé selon la revendication 1 caractérisé en ce que le catalyseur est le sel de sodium du même acide que celui qui sert à former l'anhydride.

7. Procédé selon la revendication 1 caractérisé en ce que le rapport molaire entre l'anhydride d'acide et le phénol sulfonate est d'au moins 1,2 et de préférence compris entre 1,2 et 1,3.

8. Procédé selon la revendication 1 caractérisé en ce que le rapport molaire entre le solvant aprotique polaire et le phénol sulfonate est compris entre 5 et 50.

9. Procédé selon la revendication 8 caractérisé en ce que le rapport molaire entre le solvant et le phénolsulfonate est compris entre 5 et 10.

10. Procédé selon la revendication 9 caractérisé en ce que le rapport molaire entre le solvant et le phénolsulfonate est compris entre 7 et 10.

11. Procédé selon la revendication 1 caractérisé en ce que le rapport molaire entre le catalyseur et le phénolsulfonate est supérieur à environ 0,005.

12. Procédé selon la revendication 11 caractérisé en ce que le rapport molaire entre le catalyseur et le phénolsulfonate est compris entre 0,01 et 0,02.

13. Procédé selon l'une quelconque des revendications précédentes caractérisé en ce que la température de la réaction est supérieure à 80° C et de préférence comprise entre 90 et 100° C.

14. Procédé de récupération du paraacyloxybenzènesulfonate caractérisé en ce qu'il est récupéré par relargage à l'acétone

15. Procédé selon la revendication 14 caractérisé en ce que le relargage est effectué à une température d'au moins 90° C et de préférence comprise entre 90 et 100° C.

16. Procédé selon la revendication 14 caractérisé en ce que le relargage est effectué par une quantité d'acétone environ égale à la quantité de solvant introduite.

**Patentansprüche**

1. Verfahren zur Herstellung von p-Acyloxybenzolsulfonaten der allgemeinen Formel

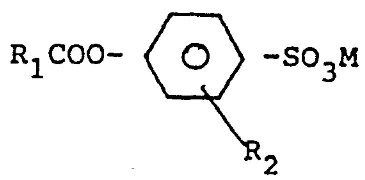

in der

- $R_1$ ein aliphatisches lineares oder verzweigtes Radikal mit 6 bis 11 Kohlenstoffatomen,
- $R_2$ ein Radikal, das aus der Gruppe, die Wasserstoff, die Halogene, die Alkylradikale mit 1 bis 4 Kohlenstoffatomen und das Radikal $SO_3M$ umfaßt, ausgewählt wird,
- M ein Alkalimetall, Erdalkalimetall oder eine Ammoniumgruppe ist,

dadurch gekennzeichnet, daß man ein Alkali-, Erdalkali- oder Ammoniumphenolsulfonat mit dem Anhydrid einer linearen oder verzweigten Säure, die 7 bis 12 Kohlenstoffatome enthält, in einem aprotischen polaren Lösungsmittel in Gegenwart einer katalytischen Menge eines Alkali- oder Erdalkalisalzes einer linearen oder verzweigten aliphatischen Säure mit 7 bis 12 C-Atomen acyliert.

2. Verfahren nach Anspruch 1, dadurch gekennzeichnet, daß das Phenolsulfonat das Natrium- oder Kaliumphenolsulfonat ist.

3. Verfahren nach Anspruch 1, dadurch gekennzeichnet, daß das Säureanhydrid das Anhydrid einer Säure mit 9 Kohlenstoffatomen ist.

4. Verfahren nach Anspruch 1, dadurch gekennzeichnet, daß das aprotische polare Lösungsmittel ausgewählt wird unter dem Dimethylformamid, dem Dimethylacetamid, dem N-Methylpyrrolidon, dem Dimethylsulfoxid und dem Sulfonlan.

5. Verfahren nach Anspruch 4, dadurch gekennzeichnet, daß das aprotische polare Lösungsmittel das Dimethylformamid ist.

6. Verfahren nach Anspruch 1, dadurch gekennzeichnet, daß der Katalysator das Natriumsalz der gleichen Säure ist, die zur Bildung des Anhydrids dient.

7. Verfahren nach Anspruch 1, dadurch gekennzeichnet, daß das molare Verhältnis zwischen dem Säureanhydrid und dem Phenolsulfonat mindestens 1,2 und vorzugsweise zwischen 1,2 und 1,3 beträgt.

8. Verfahren nach Anspruch 1, dadurch gekennzeichnet, daß das molare Verhältnis zwischen dem aprotischen polaren Lösungsmittel und dem Phenolsulfonat zwischen 5 und 50 liegt.

9. Verfahren nach Anspruch 8, dadurch gekennzeichnet, daß das molare Verhältnis zwischen dem Lösungsmittel und dem Phenolsulfonat zwischen 5 und 10 liegt.

10. Verfahren nach Anspruch 9, dadurch gekennzeichnet, daß das molare Verhältnis zwischen dem Lösungsmittel und dem Phenolsulfonat zwischen 7 und 10 liegt.

11. Verfahren nach Anspruch 1, dadurch gekennzeichnet, daß das molare Verhältnis zwischen dem Katalysator und dem Phenolsulfonat über etwa 0,005 liegt.

12. Verfahren nach Anspruch 11, dadurch gekennzeichnt, daß das molare Verhältnis zwischen dem Katalysator und dem Phenolsulfonat zwischen 0,01 und 0,02 liegt.

13. Verfahren nach einem der vorangehenden Ansprüche, dadurch gekennzeichnet, daß die Reaktionstemperatur über 80° C und vorzugsweise zwischen 90° und 100° C liegt.

14. Verfahren zur Rückgewinnung des p-Acyloxybenzolsulfonats, dadurch gekennzeichnet, daß es durch Aussalzen mit Aceton zurückgewonnen wird.

15. Verfahren nach Anspruch 14, dadurch gekennzeichnet, daß das Aussalzen bei einer Temperatur von mindestens 90° C und vorzugsweise zwischen 90° C und 100° C durchgeführt wird.

16. Verfahren nach Anspruch 14, dadurch gekennzeichnet, daß das Aussalzen mit einer Acetonmenge etwa gleich derjenigen des eingesetzten Lösungsmittels durchgeführt wird.

**Claims**

1. Process for the preparation of para-acyloxybenzenesulphonates of the following general formula:

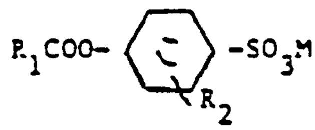

in which:

- $R_1$ is a linear or branched aliphatic radical containing 6 to 11 carbon atoms,
- $R_2$ is a radical chosen from the group comprising hydrogen, the halogens, alkyl radicals containing from 1 to 4 carbon atoms and the radical $SO_3M$,
- M is an alkali metal, alkaline-earth metal or an ammonium group, characterized in that an alkali metal, alkalineearth metal or ammonium phenolsulphonate is acylated with the anhydride of a linear or branched acid containing 7 to 12 carbon atoms, in a polar aprotic solvent in the presence of a catalytic quantity of an alkali metal or alkaline-earth metal salt of a linear or branched aliphatic acid containing 7 to 12 carbon atoms.

2. Process according to Claim 1, characterized in that the phenolsulphonate is sodium or potassium phenolsulphonate.

3. Process according to Claim 1, characterized in that the acid anhydride is the anhydride of an acid containing 9 carbon atoms.

4. Process according to Claim 1, characterized in that the polar aprotic solvent is chosen from dimethylformamide, dimethylacetamide, N-methylpyrrolidone, dimethyl sulphoxide and sulpholane.

5. Process according to Claim 5, characterized in that the polar aprotic solvent is dimethylformamide.

6. Process according to Claim 1, characterized in that the catalyst is the sodium salt of the same acid as that used to form the anhydride.

7. Process according to Claim 1, characterized in that the molar ratio of the acid anhydride to the phenolsulphonate is at least 1.2, and preferably between 1.2 and 1.3.

8. Process according to Claim 1, characterized in that the molar ratio of the polar aprotic solvent to the phenolsulphonate is between 5 and 50.

9. Process according to Claim 8, characterized in that the molar ratio of the solvent to the phenolsulphonate is between 5 and 10.

10. Process according to Claim 9, characterized in that the molar ratio of the solvent to the phenolsulphonate is between 7 and 10.

11. Process according to Claim 1, characterized in that the molar ratio of the catalyst to the phenolsulphonate is greater than approximately 0.005.

12. Process according to Claim 11, characterized in that the molar ratio of the catalyst to the phenolsulphonate is between 0.01 and 0.02.

13. Process according to any one of the preceding claims, characterized in that the reaction temperature is higher than 80° C and preferably between 90 and 100° C.

14. Process for the recovery of the para-acyloxybenzenesulphonate, characterized in that it is recovered by precipitation with acetone.

15. Process according to Claim 14, characterized in that the precipitation is carried out at a temperature of at least 90° C and preferably between 90 and 100° C.

16. Process according to Claim 14, characterized in that the precipitation is carried out by means of a quantity of acetone which is approximately equal to the quantity of solvent introduced.